# FASCICULE DE BREVET EUROPEEN

(11) **EP 3 727 664 B1**
(45) Date de publication et mention de la délivrance du brevet: **12.11.2025**
(21) Numéro de dépôt: 18815579.0
(22) Date de dépôt: 30.11.2018
(51) Int. Cl.: B01J 2/30, C07C 7/12

(54) **PROCÉDÉ DE PRÉTRAITEMENT POUR AMÉLIORER LE REMPLISSAGE D'UNE ENCEINTE AVEC DES PARTICULES SOLIDES**
VORBEHANDLUNGSVERFAHREN ZUR VERBESSERUNG DER FÜLLUNG EINER KAMMER MIT FESTSTOFFPARTIKELN
PRETREATMENT PROCESS FOR IMPROVING THE FILLING OF A CHAMBER WITH SOLID PARTICLES

(30) Priorité: 21.12.2017 FR 1762784
(43) Date de publication de la demande: 28.10.2020
(73) Titulaire: IFP Energies nouvelles, 92852 Rueil-Malmaison (FR)
(72) Inventeur: BLANCKE, Guillaume, 69340 Francheville (FR); JOSSERAND, Morgane, 69008 Lyon (FR); RICHARD, Danielle, 69003 Lyon (FR); LAROCHE, Catherine, 69390 Vernaison (FR)
(74) Mandataire: IFP Energies nouvelles
(86) Numéro de dépôt international: PCT/EP2018/083126
(87) Numéro de publication internationale: WO 2019/120938

(56) Documents cités:
- EP-A1- 1 788 594
- WO-A1-2006/094766
- DE-A1- 2 511 411
- DE-A1- 2 849 664
- FR-A1- 2 872 497
- JP-A- 2009 016 849

## Description

### DOMAINE DE L'INVENTION

L'invention concerne le domaine du remplissage des réacteurs par des particules solides. L'invention concerne plus particulièrement le chargement des réacteurs à lit fixe de type chimique ou électrochimique, pétrolier ou pétrochimique, avec des particules solides à l'état divisé, qui peuvent se présenter sous forme de billes, de grains, de cylindres, de pastilles, de bâtonnets ou de toute autre forme, mais qui sont généralement de dimensions relativement faibles. Les particules peuvent être en particulier des adsorbants, des tamis moléculaires ou des grains de catalyseurs solides, généralement extrudés, réalisés soit sous forme irrégulière, soit sous forme de bâtonnets mono- ou multilobes, dont les dimensions varient selon les cas, de quelques dixièmes de millimètres à quelques centimètres.

### ART ANTERIEUR

La plupart des procédés industriels de transformation ou de séparation impliquent l'utilisation d'un solide mis en œuvre dans un réacteur. Les performances de ces procédés sont en général directement proportionnelles au nombre de sites actifs ou de sites d'adsorption présents dans le réacteur. Il est donc souhaitable de charger le réacteur de la manière la plus dense possible. L'homme du métier connaît par exemple le chargement « Sock », consistant à déverser manuellement les particules solides à l'aide d'un conduit souple, dit "manche" ou "chaussette" (de l'anglais « sock loading »). Des techniques de chargement haute densité existent également, par exemple la technique de chargement haute densité décrite dans la demande de brevet FR2721900 A1 Catapac^{™}, permettant d'atteindre une densité de chargement d'au moins 10% supérieure au chargement de type Sock.

WO 2006/094766 A1 divulgue un procédé de prétraitement pour améliorer le remplissage d'un réacteur catalytique avec des particules de catalyseur solide.

Depuis longtemps des lubrifiants sous forme solide sont utilisés dans l'industrie pharmaceutique, l'industrie cosmétique, l'agroalimentaire et la pétrochimie (PVC, ...) dans le but d'améliorer la coulabilité des mélanges de poudres, qui est une propriété critique pour de nombreux procédés. Par exemple, dans l'industrie pharmaceutique, les granulés doivent passer par une trémie d'alimentation avant d'arriver dans le moule qui leur donnera la forme d'un comprimé. Le passage dans cette trémie doit être le plus fluide possible afin de garantir l'obtention de comprimés avec des masses homogènes. Pour ce faire, la quasi-totalité des procédés pharmaceutiques ont intégré une étape préliminaire de mélange des granulés avec un lubrifiant.

L'augmentation de la coulabilité des poudres est rendue possible grâce au lubrifiant qui va se répartir en surface des granulés. Du fait de la présence d'un film de lubrifiant formé en surface, la distance entre les granulés augmente et permet, dans un premier temps, une réduction des forces d'adhésion inter-particules.

Cependant lorsque la quantité de lubrifiant augmente de manière trop importante, il y a formation d'agrégats en surface ce qui augmente la rugosité de la surface et crée de la friction entre les particules. L'effet inverse à celui souhaité est alors observé : la force d'adhésion augmente.

Il existe donc un optimum qui est fonction de la taille des particules solides et de leur nature, car ces paramètres contrôlent les forces d'adhésion inter-particules.

Les lubrifiants industriels sont en général composés de longues chaines carbonées (au moins 14 atomes de carbone) associées à un groupe fonctionnel en bout de chaîne tel que acide carboxylique (-COOH), leur sels associés (-COO-Na+), alcool (-OH) ou ester (-COOR).

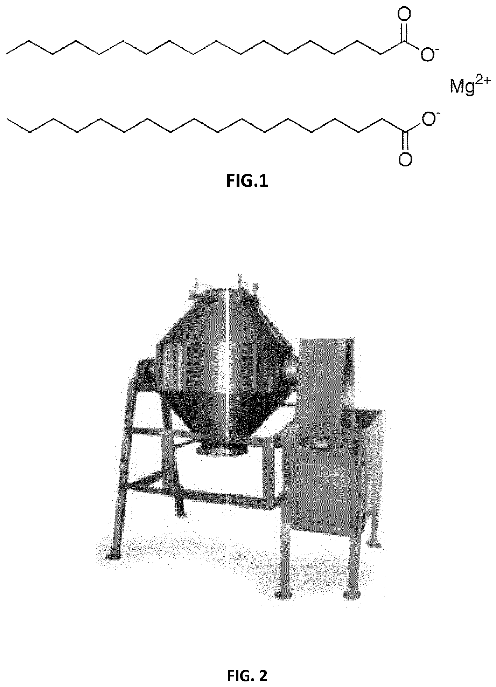

Le lubrifiant le plus utilisé le stéarate de magnésium, dont la formule est explicitée par la figure 1, dont la biocompatibilité est utile dans les comprimés pharmaceutiques, l'agroalimentaire et les cosmétiques.

Ces lubrifiants sont généralement ajoutés aux mélanges de poudre avec une étape de mélange solide-solide. Ce type de mélange est loin d'être trivial et constitue une étape capitale pour atteindre les performances souhaitées. Le lubrifiant doit être réparti de manière homogène à la surface, sans former d'agrégats, afin de réduire efficacement les forces d'adhésion inter-particules.

Dans le cadre du remplissage des réacteurs, jusqu'à présent les catalyseurs ou adsorbants sous forme de particules solides sont acheminés sous forme de gros sacs de transport ou de fûts sur site. Comme indiqué précédemment, il existe ensuite plusieurs méthodes de chargement, les principales sont :
- Le chargement manuel, de type Sock, qui consiste à transvaser manuellement les particules solides, par exemple à l'aide d'une manche ou conduit souple ;
- Le chargement à l'aide d'un dispositif interne à l'enceinte, tel que Catapac TM décrit dans le brevet FR2721900, qui permet de charger au moins 10% plus dense et de manière plus homogène au chargement Sock. Cette technique de chargement repose notamment, grâce à la configuration creuse d'un arbre de rotation, sur l'introduction et/ou le retrait d'un dispositif fonctionnel dans l'enceinte en cours de chargement. Le dispositif introduit est un dispositif comportant, à sa partie supérieure un moyen d'alimentation en particules et, à sa base située à l'intérieur de l'enceinte à charger, un système de dispersion solidaire d'un arbre central entraîné en rotation autour d'un axe sensiblement vertical par un moyen moteur, et un conduit d'alimentation entourant au moins partiellement l'arbre central, ledit arbre central est un tube de diamètre interne suffisant pour permettre de réaliser dans la zone à charger, par l'intermédiaire de ce tube et pendant la période de chargement, un certain nombre d'opérations connexes ou complémentaires à ladite opération de chargement, ce dispositif comprenant un tube fixe solidaire du conduit d'alimentation et placé à l'intérieur dudit arbre central en rotation, le tube fixe et ledit arbre central en rotation étant sensiblement coaxiaux.

Il subsiste cependant un besoin d'amélioration dans les techniques de remplissage des réacteurs par des particules solides afin d'améliorer les performances des procédés mis en œuvre dans ces réacteurs.

De manière surprenante, il a en effet été découvert que, dans certaines conditions, l'ajout d'un lubrifiant aux particules solides conduit à une augmentation des densités de remplissage des réacteurs. L'invention propose ainsi un procédé de prétraitement qui vient en amont du chargement, qu'il s'agisse d'un chargement manuel de type « Sock » ou d'un chargement haute densité, et qui permet d'améliorer le remplissage des réacteurs.

### OBJET DE L'INVENTION

L'invention concerne un procédé de prétraitement pour améliorer le remplissage d'une enceinte avec des particules solides. L'enceinte peut être tout adsorbeur, nécessitant un chargement dense de particules solides d'absorbants, connu de l'homme du métier.

### RESUME DE L'INVENTION

L'invention a pour objet un procédé de prétraitement de particules d'adsorbants de type zéolithe X pour la séparation des xylènes, pour améliorer le remplissage d'une enceinte avec lesdites particules solides, caractérisé en ce qu'on mélange lesdites particules solides avant chargement desdites particules solides dans l'enceinte avec au moins un lubrifiant qui est solide à température ambiante et choisi parmi les acides gras saturés ayant 14 atomes de carbone ou plus, les sels métalliques d'acide gras saturés ayant 14 atomes de carbone ou plus, les esters d'acides gras ayant 14 atomes de carbone ou plus, les alcools gras ayant 14 atomes de carbone ou plus, les N-alcanes linéaires ayant 16 atomes de carbone ou plus sous forme solide, l'acide fumarique, le talc, le stéaroyl fumarate de sodium, le lubrifiant étant introduit à une teneur comprise entre 0,01 et 1% par rapport au poids total du mélange de particules solides et de lubrifiant, le chargement étant effectué manuellement à l'aide d'un conduit souple ou à l'aide d'un dispositif fonctionnel interne à l'enceinte qui peut être introduit et/ou retiré de ladite enceinte.

L'invention concerne un procédé de prétraitement pour améliorer le remplissage d'une enceinte avec des particules solides, dans lequel on mélange lesdites particules solides avant chargement desdites particules solides dans l'enceinte avec au moins un lubrifiant solide à température ambiante choisi parmi les acides gras saturés ayant 14 atomes de carbone ou plus, les sels métalliques d'acide gras saturés ayant 14 atomes de carbone ou plus, les esters d'acides gras ayant 14 atomes de carbone ou plus, les alcools gras ayant 14 atomes de carbone ou plus, les N-alcanes linéaires ayant 16 atomes de carbone ou plus sous forme solide, l'acide fumarique, le talc, le stéaroyl fumarate de sodium, le lubrifiant étant introduit à une teneur comprise entre 0,01 et 1% par rapport au poids total du mélange de particules solides et de lubrifiant.

Dans un mode de réalisation, le chargement est effectué manuellement à l'aide d'un conduit souple.

Dans un autre mode de réalisation, le chargement est effectué à l'aide d'un dispositif fonctionnel interne à l'enceinte qui peut être introduit et/ou retiré de ladite enceinte.

De préférence, ledit dispositif fonctionnel interne comporte, à sa partie supérieure un moyen d'alimentation en particules et, à sa base située à l'intérieur de l'enceinte à charger, un système de dispersion solidaire d'un arbre central entraîné en rotation autour d'un axe sensiblement vertical par un moyen moteur, et un conduit d'alimentation entourant au moins partiellement l'arbre central, ledit arbre central est un tube de diamètre interne suffisant pour permettre de réaliser dans la zone à charger, par l'intermédiaire de ce tube et pendant la période de chargement, un certain nombre d'opérations connexes ou complémentaires à ladite opération de chargement, ce dispositif comprenant un tube fixe solidaire du conduit d'alimentation et placé à l'intérieur dudit arbre central en rotation, le tube fixe et ledit arbre central en rotation étant sensiblement coaxiaux.

De préférence, la teneur en lubrifiant est comprise entre 0,01 et 0,5% poids, de manière très préférée comprise entre 0,05 et 0,25% poids.

De préférence, le lubrifiant est un acide gras choisi parmi l'acide myristique, l'acide palmitique, l'acide stéarique, l'acide arachidique, l'acide béhénique, l'acide lignocérique, l'acide cérotique, ou un sel métallique d'acide gras saturés ayant 14 atomes de carbone ou plus, choisi parmi les sels à base d'aluminium, calcium, magnésium, zinc, sodium, baryum, lithium, un alcool gras ayant 14 atomes de carbone ou plus choisi parmi l'alcool myristylique, l'alcool palmitique, l'alcool stéarylique, une paraffine ayant 16 atomes de carbone ou plus, un ester d'acide gras saturés ayant 14 atomes de carbone ou plus choisi parmi les composés suivants : monostéarate de glycérol, distéarate de glycérol, monopalmitate de glycérol, dipalmitate de glycérol, tristéarate de glycérol, tripalmitate de glycérol, trimyristate de glycérol, tribenhenate de glycérol.

De manière très préférée, le lubrifiant est choisi parmi le stéarate de magnésium, le stéarate de calcium et le stéarate de baryum.

De manière encore plus préférée, le lubrifiant est le stéarate de magnésium. Avantageusement, les particules solides sont des particules de catalyseurs ou des particules d'adsorbants.

Les particules solides peuvent être sous forme de billes ou d'extrudés.

De préférence, le diamètre équivalent des particules solides est inférieur à 2 mm.

Avantageusement, les particules de lubrifiant utilisé présentent une granulométrie telle qu'au moins 90% en masse des particules de lubrifiant passe au travers d'un tamis de 90 µm. De préférence, le mélange est effectué à température ambiante et pression atmosphérique dans un mélangeur de type tambour.

De manière très préférée, le mélange est effectué avec une vitesse de rotation comprise entre 5 et 30 tr/min, pendant une durée comprise entre 5 min et 20 min, et avec un niveau de remplissage dans la cuve du mélangeur comprise entre 30% et 80%.

De préférence, l'enceinte est un réacteur de type chimique ou électrochimique, pétrolier ou pétrochimique.

### LISTE DES FIGURES

Les figures 1 à 6 illustrent l'invention à titre non limitatif.
La figure 1 représente la formule chimique développée du stéarate de magnésium.
La figure 2 représente un mélangeur tambour industriel.
Les figures 3A, 3B, 3C se rapportent à l'exemple 1 et représentent l'évolution de la densité tassée en kg d'adsorbant sec/m³ par rapport à la teneur en lubrifiant en % poids par rapport au poids total du mélange, pour trois types de lubrifiants : le stéarate de calcium, le stéarate de magnésium et le stéarate de baryum.
Les figures 4A et 4B se rapportent à l'exemple 1 et représentent l'évolution de la densité versé en kg d'adsorbant sec/m³ par rapport à la teneur en lubrifiant en % poids par rapport au poids total du mélange, pour deux types de lubrifiants : le stéarate de magnésium et le stéarate de baryum.
La figure 5 se rapporte à l'exemple 1 représente la reprise en eau en grammes d'un échantillon d'adsorbant prétraité au stéarate de magnésium conformément à l'invention, en fonction de la teneur en lubrifiant en % poids par rapport au poids total du mélange.
Les figures 6A (comparatif) et 6B (selon l'invention) se rapportent à l'exemple 1 et illustrent les propriétés anti-adhérentes d'un adsorbant prétraité avec le procédé selon l'invention.

### DESCRIPTION DETAILLEE DE L'INVENTION

Les gammes de valeurs s'entendent, sauf indication contraire, bornes incluses.

Le procédé de prétraitement selon l'invention consiste à mélanger les particules solides (par exemple sous formes de billes ou d'extrudés) avec un lubrifiant solide à température ambiante avant l'étape de chargement des particules dans l'enceinte réactionnelle afin de densifier encore plus les lits de particules. Sans vouloir être lié par une quelconque théorie, il semble que le lubrifiant, s'il est introduit dans les conditions adéquates, permet alors une réduction des forces d'adhésion inter-particules conduisant à un remplissage de meilleure qualité. L'effet observé est attribué au fait que le remplissage des réacteurs est régi par le même phénomène physique intervenant dans l'augmentation de la coulabilité, à savoir la force d'adhésion entre les particules. En effet, lorsque la force d'adhésion est faible par rapport à la force de gravité subie par la particule, la particule solide se « range » facilement et le remplissage est de bonne qualité. A l'inverse lorsque la force d'adhésion est importante devant la gravité, la particule solide a d'avantage tendance à s'agglomérer avec ses voisines, créant des espaces vides et un remplissage de mauvaise qualité.

Selon l'invention, le procédé de prétraitement proposé permet d'améliorer le remplissage d'un réacteur par des particules solides, en mélangeant les particules solides (billes ou extrudés par exemple) avec un lubrifiant solide à température ambiante avant l'étape de chargement (manuel ou à l'aide d'un dispositif fonctionnel). Le lubrifiant présent autour des particules solides est ensuite évacué dans le procédé lors du démarrage du procédé mis en œuvre dans le réacteur.

A l'échelle industrielle, le procédé de prétraitement selon l'invention peut intervenir à deux moments soit sur site, juste avant le chargement dans le réacteur, soit à la fin du procédé de préparation des particules solides d'adsorbant, une fois la mise en forme terminée et avant d'expédier les particules sur site. Le deuxième mode de réalisation peut permettre de réduire les coûts de transport en raison du meilleur tassement dans les sacs des particules et d'éviter de complexifier les procédures de démarrage.

Le lubrifiant utilisé dans le procédé de prétraitement selon l'invention est solide à température ambiante et est choisi parmi les composés suivants :
- Les acides gras saturés ayant 14 atomes de carbone ou plus
- Sels métalliques d'acide gras saturés ayant 14 atomes de carbone ou plus
- les esters d'acides gras ayant 14 atomes de carbone ou plus
- Les alcools gras ayant 14 atomes de carbone ou plus
- les N-alcanes linéaires (paraffines) ayant 16 atomes de carbone ou plus, sous forme solide.
- D'autres composés tels que l'acide fumarique, le talc, le Stéaroyl fumarate de sodium.

De préférence :
- Les acides gras saturés ayant 14 atomes de carbone ou plus sont choisis parmi les acide myristique, acide palmitique, acide stéarique, acide arachidique, acide béhénique, acide lignocérique, acide cérotique.
- Les sels métalliques d'acide gras saturés ayant 14 atomes de carbone ou plus, sont choisis parmi notamment les sels à base d'aluminium, calcium, magnésium, zinc, sodium, baryum, lithium, etc...)
- Les alcools gras ayant 14 atomes de carbone ou plus sont choisis parmi les alcools suivants : alcool myristylique, alcool palmitique, alcool stéarylique.
- Les N-alcanes linéraires sont des paraffines ayant 16 atomes de carbone ou plus, sous forme solide.
- Les esters d'acide gras saturés ayant 14 atomes de carbone ou plus sont choisis parmi les composés suivants : monostéarate de glycérol, distéarate de glycérol, monopalmitate de glycérol, dipalmitate de glycérol, tristéarate de glycérol, tripalmitate de glycérol, trimyristate de glycérol, tribenhenate de glycérol.

Parmi les composés lubrifiants préférés selon l'invention, les sels de type stéarate de magnésium, stéarate de calcium et stéarate de baryum sont préférés. De manière très préférée, on utilise le stéarate de magnésium (Figure 1).

Les lubrifiants ci-dessus peuvent être utilisés seuls ou en mélange.

Le lubrifiant utilisé dans le procédé de prétraitement selon l'invention présente avantageusement une granulométrie fine et de préférence au moins 90%masse de l'échantillon doit pouvoir passer au travers d'un tamis de 90µm. Plus particulièrement, la granulométrie du lubrifiant est telle que la taille des particules de lubrifiant est inférieure à 90 µm. La granulométrie choisie permet d'obtenir une distance optimale entre les particules solides.

Les particules solides d'adsorbant à charger dans l'enceinte ont de préférence un diamètre équivalent inférieur à 2mm.

De préférence, lesdites particules solides sont sensiblement sphériques.

La composition massique en lubrifiant dans le mélange formé par le lubrifiant et les particules solides doit être appropriée pour avoir l'effet recherché. Dans le cas d'une concentration excessive en lubrifiant, les particules de lubrifiants forment des agrégats à la surface des particules, conduisant à des distances trop importantes et à une perte de l'effet recherché. La microscopie électronique à balayage montre que la surface des particules est entièrement recouverte à partir de 1% masse en lubrifiant et qu'il y a formation d'agrégats au-delà de cette teneur.

La teneur massique en lubrifiant dans le mélange formé par le lubrifiant et les particules solides est comprise entre 0,01% et 1% poids, de manière préférée entre 0,01% et 0,5% poids, de manière plus préférée entre 0,01% et 0,25% poids, bornes incluses, par rapport au poids total du mélange formé par le lubrifiant et les particules solides.

Le mélange est avantageusement réalisé à température ambiante et pression atmosphérique dans un mélangeur de type tambour afin de maximiser les mécanismes de diffusion qui consiste en un déplacement individuel des particules de manière à ce que le lubrifiant puisse se déplacer et se répartir de manière homogène à la surface des billes.

Comme connu de l'homme du métier, (voir notamment H.Berthiaux, « Mélange et homogénéisation de solides divisés », Technique de l'ingénieur, J3397 V1 (2002)), dans ce type de mélange, on privilégie des vitesses de rotation allant de 5 à 30 tr/min pendant une durée allant de 5 min à 20 min et avec des niveaux de remplissage dans la cuve du mélangeur de 30% à 80%. Un exemple de mélangeur adapté est illustré par la figure 2. Le mélange est réalisé par la rotation de la cuve. La forme conique du mélangeur représenté sur la figure 2 assure avantageusement une homogénéisation radiale.

Les gains par rapport au chargement utilisant un dispositif de chargement haute densité, par exemple de type Catapac^{™}, sont évalués par la mesure des densités de remplissage tassées (DRT). L'analyse est réalisée par pesée de la masse de particules solides introduite dans un volume donné après tassement (2400 coups). La densité tassée représente un remplissage « maximisé » qui n'est en général pas totalement atteint par un chargement industriel haute densité. Cependant le comportement Catapac^{™} reste proche des résultats DRT et les densités industrielles sont extrapolées à partir de ces tests DRT. Cette méthode des densités de remplissage tassées est donc un indicateur cohérent des performances du remplissage Catapac^{™}.

Les gains par rapport au chargement manuel, par exemple un chargement de type « Sock » sont évalués par la mesure des densités versées. L'analyse est réalisée par pesée de la masse de particules solides introduite dans un volume donné (sans tassement). La densité versée est considérée comme équivalente à celle obtenue à l'issue d'un chargement manuel puisqu'elle résulte du même écoulement.

Ces deux méthodes permettent donc une estimation des densités obtenues à l'échelle industrielle.

Aussi, lorsque les conditions mentionnées précédemment sont réunies, le procédé de prétraitement selon l'invention permet avantageusement une augmentation des densités de remplissage en chargement manuel à l'aide d'un conduit souple pouvant aller jusqu'à 4% et une augmentation des densités de remplissage avec un dispositif interne, par exemple Catapac^{™} allant de 0 à 4%.

En plus du gain fourni sur le procédé d'application (procédé d'adsorption) inhérent à l'augmentation du nombre de sites d'adsorption dans le réacteur, on observe :
- Une diminution de l'effet de re-tassement. Il est souvent observé un re-tassement quelques heures après le démarrage de l'unité et qui correspond à une augmentation de densité de 1 à 2%. Cet effet crée un espacement vide après chargement au-dessus des lits qui peut nuire aux performances. L'étape de prétraitement selon l'invention permet de diminuer, voire de faire complètement disparaître cet effet de tassement après démarrage de l'unité.
- Une meilleure hydrodynamique dans le réacteur. L'augmentation de densité de remplissage traduit un remplissage plus homogène qui va diminuer la dispersion hydrodynamique.
- Une opération facilitée du dispositif de chargement, notamment pour les dispositifs internes de type CatapacTM TM, grâce à l'augmentation de la coulabilité du solide induit par l'étape de prétraitement.

Il a aussi été constaté que les particules solides prétraitées présentent une diminution de la reprise en eau dans le cas de particules solides hygroscopiques. Le film hydrophobe présent à la surface les protège de l'humidité et une diminution de l'électricité statique générée lors de l'écoulement.

### EXEMPLES

### Exemple 1 : Chargement d'adsorbant pour la séparation des xylènes

La séparation des xylènes est réalisée par un adsorbant de type zéolithe X sous forme de billes de diamètre nominal 0,54 mm et qui sont chargées par un chargement de type Catapac^{™} dans l'adsorbeur. Les densités industrielles sont extrapolées à partir des densités tassées (DRT).

Trois types de lubrifiants ont été testés : le stéarate de magnésium, le stéarate de calcium et le stéarate de baryum.

### Prétraitement

Au laboratoire, l'adsorbant a été mélangé avec le lubrifiant par la rotation d'un mélangeur de type tambour.

Le tambour est légèrement incliné de manière à assurer une homogénéisation radiale.

Le mode opératoire suivi est le suivant :
- Introduction de l'adsorbant dans le tambour, la quantité d'adsorbant étant d'environ 50 g
- Introduction du lubrifiant dans le tambour, dans les proportions appropriées (entre 0 et 1% en poids par rapport au poids de l'adsorbant)
- Mise en rotation du tambour à une vitesse de 10 tr/min pendant 10 min
- Mesure de la densité tassée, telle que décrite précédemment.
- Mesure de la densité versée, telle que décrite précédemment.

La teneur en eau est déterminée au début par une mesure de la perte au feu en % poids (PAF) à 900 °C puis la masse de l'échantillon est mesurée entre chaque étape, de manière à connaître la masse d'eau exacte reprise durant l'essai de prétraitement. Les essais sont réalisés dans un laboratoire à hygrométrie contrôlée et égale à 55%. Les résultats sont exprimés en masse d'adsorbant sec par unité de volume, qui est la valeur pertinente pour le procédé, en corrigeant la masse d'adsorbants lubrifiés introduits lors de la mesure de densité, de sa teneur en eau et en lubrifiant.

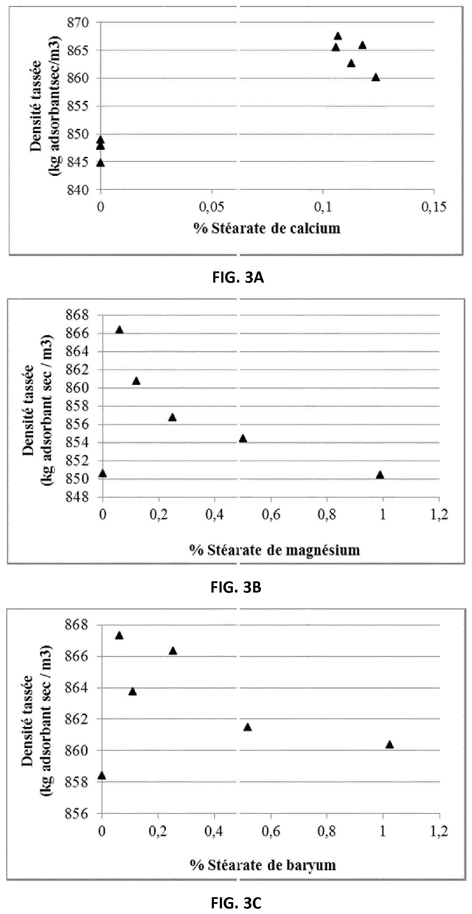

Les résultats en densité tassée pour trois lubrifiants différents (Stéarate de Magnésium, Stéarate de Calcium et Stéarate de Baryum) sont décrits par les Figures 3A, 3B et 3C. La composition du mélange est exprimée en pourcentage massique. Les points à 0% constituent la référence (comparatif), c'est-à-dire la densité de remplissage tassée de l'adsorbant seul, et donc sans mélange avec le lubrifiant.

Les résultats obtenus avec le Stéarate de Calcium (Figure 3A) montrent une reproductibilité satisfaisante et prouvent que le prétraitement permet une amélioration significative de la densité tassée.

Les tests réalisés avec le Stéarate de Baryum et de Magnésium montrent un gain en densité tassée pour l'ensemble des teneurs en lubrifiant comprises entre 0,01 et 1% par rapport au poids de mélange et révèlent un optimum pour des teneurs en lubrifiant comprises entre 0,05 et 0,25% par rapport au poids de mélange. Ce comportement est en accord avec la théorie exposée précédemment qui prévoit une diminution de la force d'adhésion dans un premier temps puis une inversion du phénomène lorsque le lubrifiant commence à s'agréger en surface, créant de la rugosité plutôt que la diminution des forces d'interactions.

Dans les conditions optimales, le procédé de prétraitement selon l'invention permet ainsi un gain en densité tassée de 2,2 % par rapport à la référence.

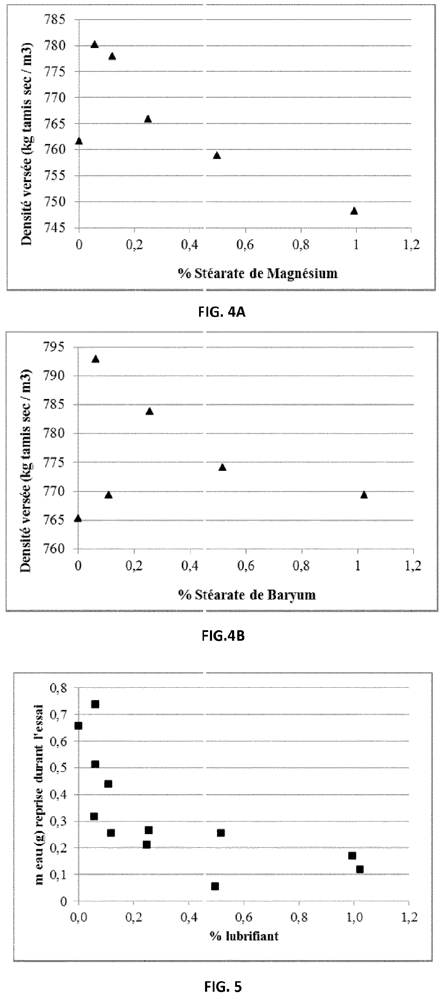

Les résultats de la densité versée sont exprimés de la même façon et sont présentés par les figures 4A (avec le stéarate de magnésium) et 4B (avec le stéarate de baryum).

L'effet du lubrifiant sur la densité versée est identique à celui observé sur la densité tassée avec une augmentation de la densité versée allant jusqu'à 3,7% par rapport à la référence.

Ce résultat confirme que le procédé de prétraitement améliore aussi bien un chargement classique manuel de type Sock (densité versée) qu'un chargement haute densité de type Catapac ^{™} (densité tassée).

Par ailleurs la mesure de l'eau reprise au cours des essais montre un rôle protecteur du lubrifiant vis-à-vis de l'humidité, tel que représenté par la figure 5 qui montre la masse d'eau en grammes reprise par l'échantillon d'adsorbant prétraité en fonction de la teneur en lubrifiant en poids par rapport à la masse totale de mélange.

Lors du démarrage d'une unité, en l'absence de procédé de prétraitement de l'adsorbant, les sacs de transport sont pesés sur site et ceux présentant une reprise en eau trop importante sont déclarés « hors spécification » et ne sont pas chargés. L'utilisation d'un prétraitement selon l'invention en amont du transport peut donc également permettre de limiter l'effet de la reprise en eau et ainsi éviter une perte avant chargement.

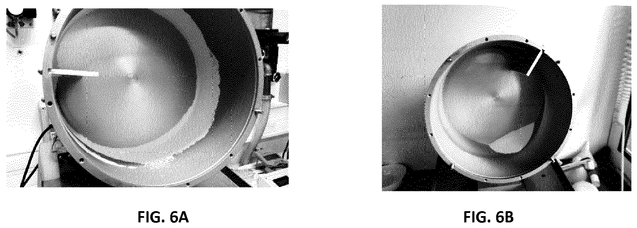

Enfin il a été remarqué que le lubrifiant possède des propriétés anti-adhérentes et réduit fortement la génération d'électricité statique. Le passage d'un adsorbant non prétraité dans le tambour aboutit à l'adhésion des particules à la paroi : les Figures 6A et 6B, illustrent ce phénomène et montrent le contenu du tambour après 10 minutes de rotation à 10 tr/min, respectivement pour l'adsorbant non prétraité et pour le mélange adsorbant-lubrifiant avec une teneur égale à 1% de stéarate de magnésium.

La présence du lubrifiant inhibe ce phénomène de génération d'électricité statique et permet une manipulation plus aisée du solide.

### Exemple 2 : Test de perçage

L'absence d'interactions du procédé de prétraitement selon l'invention avec les performances d'un procédé de séparation des xylènes a été vérifiée par des tests de perçage (chromatographie frontale) tels que décrits dans le brevet FR2903978, réalisés sur une colonne de référence comprenant de l'adsorbant (comparatif) et une colonne prétraitée comprenant de l'adsorbant prétraité avec 0,8% de Stéarate de Calcium en poids par rapport au poids total du mélange (selon l'invention). La quantité d'adsorbant utilisée pour ces tests est d'environ 75 g.

Le mode opératoire est le suivant :
- Dans le cas de la colonne pré-traitée, mélange avec 0,8% de stéarate de calcium, selon la méthodologie décrite à l'exemple A
- Remplissage de la colonne par l'adsorbant prétraité ou non et mise en place dans le banc de test.
- Remplissage par le solvant (paradiethylbenzene à température ambiante).
- Montée progressive à la température d'adsorption sous flux de solvant (5 cm³/min). Dans le cas de la colonne prétraitée, des échantillons de paradiethylbenzene sont prélevées toutes les 2 minutes pendant la montée en température.
- Injection de solvant lorsque la température d'adsorption est atteinte
- Permutation solvant/charge pour injecter la charge
- L'injection de la charge est ensuite maintenue un temps suffisant pour atteindre l'équilibre thermodynamique.
- Collecte et analyse de l'effluent du perçage.

L'opération est réalisée à un débit de charge de 10 cm³/min.

La pression est suffisante pour que la charge reste en phase liquide, soit 1 MPa. La température d'adsorption est de 175 °C. La composition de la charge est la suivante:
- Méta-xylène 45 % poids
- Para-xylène 45 % poids
- Iso-octane 10% poids (celui-ci est utilisé comme traceur pour l'estimation des volumes non-sélectifs et n'intervient pas dans la séparation)

Lors de la montée en température de la colonne prétraitée, les échantillons prélevés aux alentours de 120-130 °C deviennent bi-phasiques. Ce phénomène est attribué à la fonte du lubrifiant (sa température de fusion est de 130 °C) qui est ensuite emporté par le paradiethylbenzene.

Les résultats issus de l'analyse des courbes de perçage sont résumés dans le Tableau 1.

**Tableau 1 : Résultats des courbes de perçages**

| | Colonne Référence (comparatif) | Colonne Prétraitée (selon l'invention) |
|---|---|---|
| Perte au feu à 900°C | 5,5% | 5,5% |
| Sélectivité Para-xylène / Méta-xylène (1) | 1 | 1,003 |
| Capacité (1) | 1 | 0,995 |
| Hauteur plateau théorique (1) | 1 | 1,011 |

| | | |
|---|---|---|
| (1) Les résultats sont exprimés en relatif par rapport à la colonne de référence, c'est-à-dire en divisant la valeur obtenue par la valeur obtenue avec la colonne de référence. | | |

On constate que le prétraitement n'a pas d'impact sur les performances brutes de l'adsorbant. Ces résultats confirment l'absence d'interactions avec les performances du procédé et confirment le départ du lubrifiant avec le solvant (ici le paradiethylbenzene) lors du démarrage de l'unité d'adsorption des xylènes.

Par conséquent, l'exemple montre que l'application du procédé de prétraitement selon l'invention au chargement d'un adsorbant dans un procédé de séparation des xylènes permet d'améliorer la densité de chargement dense type Catapac^{™} de 2,2%, sans altérer les performances de l'adsorbant, ce qui conduit à une augmentation de la productivité du procédé de 2,2 % (proportionnelle à la masse d'adsorbant présent dans le réacteur).

Le procédé de prétraitement testé dans le cadre de la séparation des xylènes permet une augmentation des densités tassées et versées de 2 à 4 %, sans altérer les performances de l'adsorbant. Etant donné que les densités industrielles sont extrapolées à partir des densités tassées et que les performances sont proportionnelles à la masse d'adsorbant présent dans le réacteur, le procédé de prétraitement selon l'invention permet à lui seul une augmentation de productivité de 2 à 4% du procédé de séparation des xylènes.

### AVANTAGES DU PROCEDE SELON L'INVENTION

Outre le gain en densité de chargement des particules solides, le procédé de prétraitement de particules solides selon l'invention permet également :
- Une diminution de l'effet de re-tassement des lits après le démarrage
- Une diminution de la dispersion hydrodynamique
- Une opération facilitée du chargement en raison de la coulabilité améliorée
- Une diminution de la reprise en eau des particules solides
- Une diminution de l'électricité statique générée lors de l'écoulement

Le procédé de prétraitement selon l'invention s'applique plus particulièrement au chargement d'adsorbants pour la mise en œuvre d'un procédé de séparation des xylènes, par exemple le procédé ELUXYL ^{™}. Quel que soit le type de chargement, le procédé de prétraitement testé dans le cadre de la séparation des xylènes permet une augmentation nette de la densité de chargement, sans altérer les performances intrinsèques de l'adsorbant. Les performances du procédé de séparation des xylènes étant proportionnelles à la masse d'adsorbant présent dans le réacteur, le procédé de prétraitement selon l'invention permet à lui seul une augmentation de productivité du procédé de séparation des xylènes.

## Revendications

1. Procédé de prétraitement de particules d'adsorbants de type zéolithe X pour la séparation des xylènes, pour améliorer le remplissage d'une enceinte avec lesdites particules solides, **caractérisé en ce qu'**on mélange lesdites particules solides avant chargement desdites particules solides dans l'enceinte avec au moins un lubrifiant qui est solide à température ambiante et choisi parmi les acides gras saturés ayant 14 atomes de carbone ou plus, les sels métalliques d'acide gras saturés ayant 14 atomes de carbone ou plus, les esters d'acides gras ayant 14 atomes de carbone ou plus, les alcools gras ayant 14 atomes de carbone ou plus, les N-alcanes linéaires ayant 16 atomes de carbone ou plus sous forme solide, l'acide fumarique, le talc, le stéaroyl fumarate de sodium, le lubrifiant étant introduit à une teneur comprise entre 0,01 et 1% par rapport au poids total du mélange de particules solides et de lubrifiant, le chargement étant effectué manuellement à l'aide d'un conduit souple ou à l'aide d'un dispositif fonctionnel interne à l'enceinte qui peut être introduit et/ou retiré de ladite enceinte.

2. Procédé selon la revendication 1 dans lequel ledit dispositif fonctionnel interne comporte, à sa partie supérieure un moyen d'alimentation en particules et, à sa base située à l'intérieur de l'enceinte à charger, un système de dispersion solidaire d'un arbre central entraîné en rotation autour d'un axe sensiblement vertical par un moyen moteur, et un conduit d'alimentation entourant au moins partiellement l'arbre central, ledit arbre central est un tube de diamètre interne suffisant pour permettre de réaliser dans la zone à charger, par l'intermédiaire de ce tube et pendant la période de chargement, un certain nombre d'opérations connexes ou complémentaires à ladite opération de chargement, ce dispositif comprenant un tube fixe solidaire du conduit d'alimentation et placé à l'intérieur dudit arbre central en rotation, le tube fixe et ledit arbre central en rotation étant sensiblement coaxiaux.

3. Procédé selon l'une des revendications précédentes dans lequel la teneur en lubrifiant est comprise entre 0,01 et 0,5% poids.

4. Procédé selon la revendication 3 dans lequel la teneur en lubrifiant est comprise entre 0,05 et 0,25% poids.

5. Procédé selon l'une des revendications précédentes dans lequel le lubrifiant est un acide gras choisi parmi l'acide myristique, l'acide palmitique, l'acide stéarique, l'acide arachidique, l'acide béhénique, l'acide lignocérique, l'acide cérotique, ou un sel métallique d'acide gras saturés ayant 14 atomes de carbone ou plus, choisi parmi les sels à base d'aluminium, calcium, magnésium, zinc, sodium, baryum, lithium, un alcool gras ayant 14 atomes de carbone ou plus choisi parmi l'alcool myristylique, l'alcool palmitique, l'alcool stéarylique, une paraffine ayant 16 atomes de carbone ou plus, un ester d'acide gras saturés ayant 14 atomes de carbone ou plus choisi parmi les composés suivants : monostéarate de glycérol, distéarate de glycérol, monopalmitate de glycérol, dipalmitate de glycérol, tristéarate de glycérol, tripalmitate de glycérol, trimyristate de glycérol, tribenhenate de glycérol.

6. Procédé selon l'une des revendications précédentes dans lequel le lubrifiant est choisi parmi le stéarate de magnésium, le stéarate de calcium et le stéarate de baryum.

7. Procédé selon la revendication 6 dans lequel le lubrifiant est le stéarate de magnésium.

8. Procédé selon l'une des revendications précédentes dans lequel les particules solides sont sous forme de billes ou d'extrudés.

9. Procédé selon l'une des revendications précédentes dans lequel le diamètre équivalent des particules solides est inférieur à 2 mm.

10. Procédé selon l'une des revendications précédentes dans lequel les particules de lubrifiant utilisé présentent une granulométrie telle qu'au moins 90% en masse des particules de lubrifiant passe au travers d'un tamis de 90 µm.

11. Procédé selon l'une des revendications précédentes dans lequel le mélange est effectué à température ambiante et pression atmosphérique dans un mélangeur de type tambour.

12. Procédé selon la revendication 11 dans lequel le mélange est effectué avec une vitesse de rotation comprise entre 5 et 30 tr/min, pendant une durée comprise entre 5 min et 20 min, et avec un niveau de remplissage dans la cuve du mélangeur comprise entre 30% et 80%.

13. Procédé selon l'une des revendications précédentes dans lequel l'enceinte est un réacteur de type chimique ou électrochimique, pétrolier ou pétrochimique.

## Patentansprüche

1. Verfahren zur Vorbehandlung von Partikeln von Adsorbenzien vom Typ Zeolith X zur Trennung der Xylene, um die Füllung einer Kammer mit den Feststoffpartikeln zu verbessern, **dadurch gekennzeichnet, dass** die Feststoffpartikel vor dem Einfüllen der Feststoffpartikel in die Kammer mit mindestens einem Schmiermittel gemischt werden, das bei Umgebungstemperatur fest ist und aus gesättigten Fettsäuren mit 14 oder mehr Kohlenstoffatomen, Metallsalzen von gesättigten Fettsäuren mit 14 oder mehr Kohlenstoffatomen, Estern von Fettsäuren mit 14 oder mehr Kohlenstoffatomen, Fettalkoholen mit 14 oder mehr Kohlenstoffatomen, linearen N-Alkanen mit 16 oder mehr Kohlenstoffatomen in fester Form, Fumarsäure, Talkum, Natriumstearylfumarat ausgewählt ist, wobei das Schmiermittel mit einem Gehalt zwischen 0,01 und 1 % bezogen auf das Gesamtgewicht der Mischung aus Feststoffpartikeln und Schmiermittel eingeführt wird, wobei das Befüllen manuell mithilfe einer flexiblen Leitung oder mithilfe einer internen funktionellen Vorrichtung der Kammer, die in die Kammer eingeführt und/oder aus ihr entnommen werden kann, durchgeführt wird.

2. Verfahren nach Anspruch 1, wobei die interne funktionelle Vorrichtung an ihrem oberen Teil eine Partikelzuführeinrichtung und an ihrer im Inneren der zu befüllenden Kammer gelegenen Basis ein Dispersionssystem, das mit einer zentralen Welle fest verbunden ist, die um eine im Wesentlichen vertikale Achse durch eine Motoreinrichtung drehangetrieben wird, und eine Zuführleitung, welche die zentrale Welle mindestens teilweise umgibt, umfasst, wobei die zentrale Welle ein Rohr mit einem Innendurchmesser ist, der ausreicht, um zu ermöglichen, in dem zu befüllenden Bereich über dieses Rohr und während des Befüllzeitraums eine bestimmte Anzahl von Vorgängen auszuführen, die mit dem Befüllvorgang zusammenhängen oder diesen ergänzen, wobei diese Vorrichtung ein feststehendes Rohr umfasst, das mit der Zuführleitung fest verbunden und im Inneren der drehenden zentralen Welle platziert ist, wobei das feststehende Rohr und die drehende zentrale Welle im Wesentlichen koaxial sind.

3. Verfahren nach einem der vorhergehenden Ansprüche, wobei der Gehalt an Schmiermittel zwischen 0,01 und 0,5 Gew.-% beträgt.

4. Verfahren nach Anspruch 3, wobei der Gehalt an Schmiermittel zwischen 0,05 und 0,25 Gew.-% beträgt.

5. Verfahren nach einem der vorhergehenden Ansprüche, wobei das Schmiermittel eine Fettsäure ist, die aus Myristinsäure, Palmitinsäure, Stearinsäure, Arachinsäure, Behensäure, Lignocerinsäure, Cerotinsäure ausgewählt ist, oder ein Metallsalz von gesättigten Fettsäuren mit 14 oder mehr Kohlenstoffatomen, das aus den Salzen auf Basis von Aluminium, Calcium, Magnesium, Zink, Natrium, Barium, Lithium ausgewählt ist, ein Fettalkohol mit 14 oder mehr Kohlenstoffatomen, der aus Myristylalkohol, Palmitylalkohol, Stearylalkohol ausgewählt ist, ein Paraffin mit 16 oder mehr Kohlenstoffatomen, ein Ester von gesättigten Fettsäuren mit 14 oder mehr Kohlenstoffatomen, der aus den folgenden Verbindungen ausgewählt ist: Glycerolmonostearat, Glyceroldistearat, Glycerolmonopalmitat, Glyceroldipalmitat, Glyceroltristearat, Glyceroltripalmitat, Glyceroltrimyristat, Glyceroltribehenat.

6. Verfahren nach einem der vorhergehenden Ansprüche, wobei das Schmiermittel aus Magnesiumstearat, Calciumstearat und Bariumstearat ausgewählt ist.

7. Verfahren nach Anspruch 6, wobei das Schmiermittel Magnesiumstearat ist.

8. Verfahren nach einem der vorhergehenden Ansprüche, wobei die Feststoffpartikel in Form von Kugeln oder Extrudaten vorliegen.

9. Verfahren nach einem der vorhergehenden Ansprüche, wobei der äquivalente Durchmesser der Feststoffpartikel weniger als 2 mm beträgt.

10. Verfahren nach einem der vorhergehenden Ansprüche, wobei die Partikel des verwendeten Schmierstoffs eine derartige Korngröße aufweisen, dass mindestens 90 Massen-% der Schmierstoffpartikel durch ein Sieb mit 90 µm fallen.

11. Verfahren nach einem der vorhergehenden Ansprüche, wobei das Mischen bei Umgebungstemperatur und atmosphärischem Druck in einem Trommelmischer durchgeführt wird.

12. Verfahren nach Anspruch 11, wobei das Mischen mit einer Drehzahl zwischen 5 und 30 U/min, während einer Dauer zwischen 5 min und 20 min und mit einem Füllgrad im Behälter des Mischers zwischen 30 % und 80 % durchgeführt wird.

13. Verfahren nach einem der vorhergehenden Ansprüche, wobei die Kammer ein chemischer oder elektrochemischer, petrochemischer oder Ölreaktor ist.

## Claims

1. Process for pretreatment of X zeolite type adsorbent particles for the separation of xylenes, for improving the filling of a chamber with said solid particles, **characterized in that** said solid particles are mixed before loading of said solid particles into the chamber with at least one lubricant that is solid at ambient temperature and chosen from saturated fatty acids having 14 or more carbon atoms, metal salts of saturated fatty acids having 14 or more carbon atoms, esters of fatty acids having 14 or more carbon atoms, fatty alcohols having 14 or more carbon atoms, linear N-alkanes having 16 or more carbon atoms in solid form, fumaric acid, talc, sodium stearoyl fumarate, the lubricant being introduced at a content of between 0.01% and 1% relative to the total weight of the mixture of solid particles and lubricant, the loading being carried out manually with the aid of a flexible hose or with the aid of a functional device internal to the chamber which may be introduced into and/or withdrawn from said chamber.

2. Process according to Claim 1, in which said internal functional device comprises, in its upper part, a means for feeding with particles and, in its base located inside the chamber to be loaded, a dispersion system attached to a central shaft rotated about a substantially vertical axis by a motor means, and a feed pipe at least partially surrounding the central shaft, said central shaft is a tube having an internal diameter sufficient to make it possible to carry out, in the zone to be loaded, by means of this tube and during the loading period, a certain number of operations connected or complementary to said loading operation, this device comprising a stationary tube attached to the feed pipe and placed inside said rotary central shaft, the stationary tube and said rotary central shaft being substantially coaxial.

3. Process according to one of the preceding claims, in which the lubricant content is between 0.01% and 0.5% by weight.

4. Process according to Claim 3, in which the lubricant content is between 0.05% and 0.25% by weight.

5. Process according to one of the preceding claims, in which the lubricant is a fatty acid chosen from myristic acid, palmitic acid, stearic acid, arachidic acid, behenic acid, lignoceric acid or cerotic acid, or a metal salt of saturated fatty acids having 14 or more carbon atoms, chosen from salts based on aluminium, calcium, magnesium, zinc, sodium, barium or lithium, a fatty alcohol having 14 or more carbon atoms chosen from myristyl alcohol, palmityl alcohol or stearyl alcohol, a paraffin having 16 or more carbon atoms, an ester of saturated fatty acids having 14 or more carbon atoms chosen from the following compounds: glyceryl monostearate, glyceryl distearate, glyceryl monopalmitate, glyceryl dipalmitate, glyceryl tristearate, glyceryl tripalmitate, glyceryl trimyristate or glyceryl tribenhenate.

6. Process according to one of the preceding claims, in which the lubricant is chosen from magnesium stearate, calcium stearate and barium stearate.

7. Process according to Claim 6, in which the lubricant is magnesium stearate.

8. Process according to one of the preceding claims, in which the solid particles are in the form of beads or extrudates.

9. Process according to one of the preceding claims, in which the equivalent diameter of the solid particles is less than 2 mm.

10. Process according to one of the preceding claims, in which the particles of lubricant used have a particle size such that at least 90% by weight of the particles of lubricant pass through a 90 µm screen.

11. Process according to one of the preceding claims, in which the mixing is carried out at ambient temperature and atmospheric pressure in a drum-type mixer.

12. Process according to Claim 11, in which the mixing is carried out with a speed of rotation of between 5 and 30 rpm, for a time of between 5 min and 20 min, and with a degree of filling in the tank of the mixer of between 30% and 80%.

13. Process according to one of the preceding claims, in which the chamber is a chemical or electrochemical, petroleum or petrochemical type reactor.
